# EUROPEAN PATENT APPLICATION

(11) **EP 2 818 553 A2**
(43) Date of publication of application: **31.12.2014**
(21) Application number: 14173364.2
(22) Date of filing: 21.06.2014
(51) Int. Cl.: C12P 7/62

(54) **Method for converting polyethylene to biodegradable polyhydroxyalkanoate**

(30) Priority: 24.06.2013 GB 201311177
(71) Applicant: UNIVERSITY COLLEGE DUBLIN, Dublin 4 (IE); The Provost, Fellows, Foundation Scholars, & the other members of Board, of the College of the Holy & Undiv. Trinity of Queen Elizabeth near Dublin, Dublin 2 (IE)
(72) Inventor: O'Connor, Kevin, Dublin, D1 (IE); Guzik, Maciej, Dublin, D4 (IE); Padamati, Ramesh, Dublin, D1 (IE); Kenny, Shane, Bray, Co. Wicklow (IE)
(74) Representative: Purdy, Hugh Barry

(57) **Abstract**

A method for producing polyhydroxyalkanoate (PHA) comprises the steps of culturing in a culture medium comprising pyrolysis wax obtained from the pyrolysis of polyethylene and optionally a surfactant one or more bacterial strains which are capable of accumulating PHA from pyrolysis wax, and recovering the PHA from the culture medium. Typically, the bacterial strains are selected from the group consisting of: A. *calcoaceticus* BD413; A. *calcoaceticus* RR8; *B. sepacia* RR10; *P. aeruginosa* 3924; *P*. *aeruginosa* GL-1; *P. aeruginosa* PAO1; *P. aeruginosa* RR1; and *P. olevorans.*

## Description

### Technical Field

The invention relates to a method of converting polyethylene to biodegradable polyhydroxyalkanoate.

### Background to the Invention

Petrochemical based plastics, produced worldwide on a multimillion tonne scale, pervade modem society as a result of their versatile and highly desirable properties. Polyethylene (PE) is produced more than any other polymer type, making up 29.1 % of worldwide polymer production.. PE has a broad range of physico-chemical proprieties, which enable its use in a variety of products. These include heavy-duty commodities, such as water pipes, food containers, toys and detergent/chemicals storage containers. PE is also widely used in film applications such as plastic bags, agricultural films, bubble wraps or multilayer and composite films. Like other petrochemical plastics the success of PE as a convenience bulk commodity polymer has led to post consumer PE products becoming a major waste problem. Current recycling technologies focus mainly on mechanical recycling and energy recovery. However, these technologies recycle PE to low value products (A1-Salem, *et al.,* 2009) or allow for a quick end to the carbon cycle by incineration (Butler, *et al*., 2011).
The conversion of other petrochemical polymers, specifically polystyrene and polyethylene terephthalate to polyhydroxyalkanoates has already been described (Ward, *et al*., 2006, Kenny, *et al*., 2008)

### Statements of Invention

The invention is based on the finding that polyethylene (PE) pyrolysis wax, a by-product obtained in the process for converting PE to biodiesel, can act as a substrate for the bacterial accumulation of polyhydroxyalkanoate (PHA). Bacteria capable of growing on PE pyrolysis wax, and accumulating PHA, are not described in the literature. Two papers, Yakimov and Sabriova, describe a strain (*Alkanivorax borkumensis* SK2 - DSMZ 11573) that is capable of growing on a broad range of hydrocarbon chains (C8-C32), however the Applicant discovered that this strain cannot grow on PE pyrolysis wax, despite it having a similar (C8-C32) hydrocarbon chain profile. Despite this, the Applicant has surprisingly discovered a number of strains that can grow on PE pyrolysis wax, and accumulate PHA, (Table 1 and 2), namely: *Acinetobacter calcoaceticus* BD413 (Juni & Janik, 1969 - American Type Culture Collection ATCC 33305);
*Acinetobacter calcoaceticus* RR8 (Yuste, *et al*., 2000);
*Burkholderia. sepacia* RR10 (Yuste et al 2000);
*Pseudomonas. aeruginosa* 3924 (Hori, *et al.,* 2002 - National Insttitute of Technology and Evaluation, NBRC Culture Collection, Japan, NBRC 3924);
*Pseudomonas. aeruginosa* GL-1 (Arino, *et al*., 1998 - Collection of Institute Pasteur CIP 104590);
*Pseudomonas. aeruginosa* PAO1 (Stover, *et al*., 2000 - American Type Culture Collection ATCC 47085);
*Pseudomonas aeruginosa* RR1 (Yuste et al, 2000); and
*Pseudomonas olevorans* (Freitas, *et al*., 2007 - Agricultural Research Service Culture Collection NRRL B14682).

For some of the strains, namely A. *calcoaceticus* BD413; A. *calcoaceticus* RR8; *B. sepacia* RR10; *P. aeruginosa* PAO1; and *P. aeruginosa* RR1, while the strains can grow on PE pyrolysis wax, they require the presence of a surfactant, typically a rhamnolipid, in the culture medium in order to accumulate PHA (Table 2).

Thus, in a first aspect, the invention provides a method for producing polyhydroxyalkanoate (PHA), comprising the step of:
- culturing in a culture medium comprising pyrolysis wax obtained from the pyrolysis of PE (hereafter "PE pyrolysis wax") and optionally a surfactant one or more bacterial strains which are capable of accumulating PHA from pyrolysis wax; and
- recovering the PHA from the bacterial biomass.

In a further aspect, the invention provides a method for producing polyhydroxyalkanoate (PHA) from polyethylene (PE), comprising the steps of:
- subjecting the polyethylene to pyrolysis under suitable conditions for producing a liquid diesel fraction and a PE pyrolysis wax fraction, and
- producing PHA from the PE pyrolysis wax according to a method of the invention.

The invention also relates to PHA obtained according to a method of the invention.

Preferably, the or each bacterial strain capable of accumulating PHA from PE pyrolysis wax is selected from the group consisting of: *A*. *calcoaceticus* BD413; *A*. *calcoaceticus* RR8; *B. sepacia* RR10; *P. aeruginosa* 3924; *P. aeruginosa* GL-1; *P. aeruginosa* PAO1; *P. aeruginosa* RR1; and *P*. *olevorans.*

In one embodiment, the or each bacterial strain is selected from *P. aeruginosa* GL-1 and *P. olevorans.* With these strains, the PE pyrolysis wax may be the sole energy or carbon source. Preferably, the culture medium comprises a surfactant, typically a biosurfactant, and ideally a rhamnolipid.

In another embodiment, the culture medium comprises a surfactant in addition to the pyrolysis wax, and wherein the or each bacterial strain is selected from the group consisting of: *A*. *calcoaceticus* BD413; *A*. *calcoaceticus* RR8; *B. sepacia* RR10; *P. aeruginosa* PAO1; *P. aeruginosa* RR1.

Typically, the surfactant is a biosurfactant, and ideally is a rhamnolipid. Typically, the culture medium comprises 0.01% to 0.1%, 0.03% to 0.07%, and ideally about 0.05% surfactant (w/v). Ideally, the culture medium comprises exogenous rhamnolipid. Suitably, the rhamnolipid is obtained from *P. aeruginosa* GL-1.

Typically, the culture medium comprises an inorganic nitrogen source, for example an ammonium salt. Preferably, the ammonium salt is ammonium chloride or ammonium nitrate. Suitably, the culture medium comprises 0.1 to 1.0g/L, 0.2 to 0.3g/L ammonium salt, preferably ammonium nitrate.

Typically, the culture medium comprises 0.01 % to 5%, 1% to 5%, 1% to 3%, or ideally about 2%, pyrolysis wax (w/v).

In one embodiment, the culture medium comprises 0.01% to 5.0% pyrolysis wax (w/v), an ammonium salt in an amount of 0.1% to 1.0%, and optionally a rhamnolipid in an amount of 0.01 % to 0.1 % (w/v).

In another embodiment, the culture medium comprises 1% to 5.0% pyrolysis wax (w/v), an ammonium salt in an amount of 0.2 to 0.3g/L, and optionally a rhamnolipid in an amount of 0.03% to 0.7% (w/v).

Preferably, the culture medium comprises 0.01% to 5.0% pyrolysis wax (w/v), and an ammonium salt in an amount of 0.1% to 1.0%, in which the bacterial strains are selected from *P*. *aeruginosa* GL-1 and *P*. *olevorans.*

Alternatively, the culture medium comprises 0.01% to 5.0% pyrolysis wax (w/v), an ammonium salt in an amount of 0.1% to 1.0%, and a rhamnolipid in an amount of 0.01% to 0.1% (w/v), and wherein the bacterial strains are selected from the group consisting of: *A*. *calcoaceticus* BD413; *A*. *calcoaceticus* RR8; *B. sepacia* RR10; *P. aeruginosa* GL-1; *P. aeruginosa* PAO1; *P. aeruginosa* RR1; and *P*. *olevorans.*

Preferably, the one or more strains of bacteria are cultured in the culture medium for a period of 12-96 hours, suitably from 12-72, and generally about 20-60 hours, and the temperature of the culture medium is suitably maintained at about 25°C to 37°C, typically about 30°C.

Preferably, the culture medium is a minimum salt medium, ideally nitrogen limited with a preferable maximum nitrogen content of 0.5g/L.

Suitably, the step of recovering PHA from the culture medium comprises:
- harvesting the cells from the culture medium by centrifugation to produce a pellet of cells
- freeze-drying the pelleted cells;
- resuspending the dried material in non-aqueous solvent (preferably acetone); centrifuging the supernatant and retaining the supernatant;
- filtering the supernatant; and
- evaporating solvent from the filtrate to provide a suspension of PHA.

### Brief Description of the Figures

**Fig. 1****:** Monomer composition of mcl-PHA accumulated from PE derived pyrolysis wax.
**Fig. 2****:** Utilisation of PE pyrolysis wax by *P. aeruginosa* GL-1 in a shake flask over a 48 hour period with a starting concentration of 1.95g_{c}L⁻¹
**Fig. 3****:** Comparison of growth and PHA accumulation by *P. aeruginosa* GL-1 and PAO1 with 2% w/v PE pyrolysis wax in shake flasks supplied with different inorganic nitrogen sources (4A and 4B) and in the presence of 0.05% w/v rhamnolipids (4C). Cell dry weight (CDW) (g L⁻¹, ▲), PHA content of cells (% of CDW,●) were tracked over time. Conditions: Panel A - 0.2 g L⁻¹ ammonium chloride as nitrogen source; Panel B - 0.2 g L⁻¹ ammonium nitrite as nitrogen source; Panel C - 0.2 g L⁻¹ ammonium nitrite and 0.05 % rhamnolipids.

### Detailed Description of the Invention

Broadly, the invention relates to a method for converting non-biodegradable polyethylene to biodegradable polyhydroxyalkanoate. The method involves performing pyrolysis on the PE to generate a liquid biodiesel fraction and a waxy by-product known as PE pyrolysis wax. The PE pyrolysis wax is then used as a substrate in a bacterial culture medium comprising certain strains of bacteria capable of metabolising the PE pyrolysis wax and accumulating PHA. The bacteria capable of metabolising PE pyrolysis wax include *A*. *calcoaceticus* BD413; A. *calcoaceticus* RR8; *B. sepacia* RR10; *P. aeruginosa* 3924; *P. aeruginosa* GL-1; *P. aeruginosa* PAO1; *P. aeruginosa* RR1; and *P. olevorans.* Two of these strain are capable of growth and accumulation of PHA using PE pyrolysis wax as the sole energy source. For the remaining strains, a surfactant, preferably a rhamnolipid, must be added to the culture broth to achieve both bacterial growth and PHA accumulation.

In this specification, the term "polyhydroxyalkanoate" or "PHA" are used interchangeably and refer to a range of biodegradable polymers that consist of polyesters of (R)-3-hydroxyalkanoic acids.

In this specification, the term "polyethylene" or "PE" are used interchangeably and refer to a non-biodegradable thermoplastic polymer generally having the chemical formula (C₂-H₄)ₙH₂.

In this specification, the term "pyrolysis" refers to the process of thermochemical decomposition of matter in the absence of oxygen. It is generally achieved by heating in the absence of oxygen to a temperature of at least 300°C, and often much higher, resulting in the long chain hydrocarbons being converted to shorter chain hydrocarbons, and a great increase in the elemental carbon content of the matter. Specific methods of performing pyrolysis on PE to produce biodiesel and PE pyrolysis wax are described in (Conesa, *et al.,* 1994, Wallis & Bhatia, 2007, Aguado, *et al.,* 2009, Rasul Jan, *et al.,* 2010, Kumar, *et al.,* 2011).

The term "surfactant" refers to an organic compound that is amphiphilic, having both a hydrophilic domain and a hydrophobic domain. In particular, the term refers to a biosurfactant which is a surfactant produced by a living cell, particularly microbial cells such as bacteria. Most biosurfactants are glycolipids, comprising a carbohydrate attached to a long aliphatic side chain, whereas others like lipopeptides and lipoproteins are more complex. Examples of biosurfactants include rhamnolipids (produced by *P. aeruginosa*) and surfactins (produced by *Bacillus ssp*.).

### Experimental Section

### PE pyrolysis wax

Polyethylene pyrolysis product (PE pyrolysis wax) was supplied by Cynar plc Ltd, Portlaoise, Ireland and was generated from waste agricultural PE films.

### Bacterial strains and growth medium

The strains employed were obtained from The Collection of Institut Pasteur (CIP), German Collection of Microorganisms and Cell Cultures (DSMZ), Agricultural Research Service Culture Collection (NRRL), American Type Culture Collection (ATCC), National Institute of Technology and Evaluation, Japan (NBRC) and private culture collection of F. Rojo. The minimal salt medium (MSM) was prepared as previously described (Schlegel, *et al*., 1961) and used as the growth medium for all of the culture techniques discussed here. Strains were maintained on MSM solidified with 15 g L⁻¹ and supplemented with 20 mM sodium gluconate as a carbon source. Pyrolysed polyethylene wax (PE pyrolysis wax) was used as a carbon source in all In this specification, the term "polyhydroxyalkanoate" experiments.

### Rhamnolipid production and supplementation to shake flask cultures

Cultures were supplemented with 0.05% w/v rhamnolipids produced by *Pseudomonas aeruginosa* GL-1 strain as described by Arino, *et al.* (1998). Briefly, *P. aeruginosa* GL-1 was grown in a synthetic medium supplemented with 30 g L⁻¹ of glycerol for 5 days. Supernatant was clarified by centrifugation, deproteinised at 100°C for 10 minutes and acidified to pH 2 with 6M HCl. This was followed by an overnight extraction of rhamnolipids with ethyl acetate. Organic solvent was evaporated under reduced pressure in order to obtain a honey-like rhamnolipid mixture. Composition of rhamnolipids produced was determined accordingly to Arino, *et al.* (1998) by means of TLC and GC-MS methods. This crude extract was supplemented to the bacterial cultures at a concentration of 500 mg L⁻¹ (0.05% w/v).

### Growth conditions for PHA accumulation

For screening purposes, all strains were grown in a 250 mL Erlenmeyer flask containing 50 mL of nitrogen limited MSM medium and various carbon sources at a final concentration of 1.95 gram of carbon per litre (gc L⁻¹ = 0.05% w/v). The flasks were incubated at 30°C with shaking at 250 rpm for 48 hours. To screen for organisms capable of PHA accumulation the inorganic nitrogen source ammonium chloride (NH₄Cl) was limited to 0.25 g L⁻¹ (65 mgN L⁻¹ = 4.6 mM). In order to establish effect of carbon concentration on growth and PHA accumulation, PE pyrolysis wax concentration was increased from 0.05% to 2% w/v and incubation time was extended up to 6 days. In addition we investigated the effect of two different nitrogen sources (NH₄Cl and (NH₄)₂SO₄) on growth and PHA accumulation.

### PHA content and monomer composition determination from bacterial cultures

The polymer content of lyophilized whole bacterial cells was determined by subjecting cells to acidic methanolysis according to previously described protocols (Brandl, *et al.,* 1988, Lageveen, *et al*., 1988). The 3-hydroxyalkanoic acid methyl esters were assayed by gas chromatography (GC) using an Agilent 6890N chromatograph equipped with a BP21 capillary column (25 m x 0.25 mm x 0.32 µm; SGE Analytical Sciences) and a flame ionization detector (FID) with a temperature program of 120°C for 5 min; followed by ramp of 3°C min⁻¹ until 180°C held for 10 min. For the peak identification, commercially available 3-hydroxyalkanoic acids were methylated as described above for PHA samples. PHA monomer determination was confirmed using an Agilent 6890N GC fitted with a 5973 series inert mass spectrophotometer (MS), a HP-5 column (12 m x 0.2 mm x 0.33 µm; Hewlett-Packard) was used with an oven method of 50°C for 3 min, increasing by 10 °C min⁻¹ to 250°C and holding for 1 min.

### Nitrogen determination assays

The concentration of nitrogen in the growth media was monitored over time using the previously described indophenol method (Scheiner, 1976).

### Determination of hydrocarbon utilisation during growth

Bacterial cultures grown in liquid media on PE pyrolysis wax were extracted at various time points with chloroform in order to track the substrate utilisation. The organic layer was analysed on an Agilent 6890N series GC fitted with a 5 m x 0.53 mm x 0.15 µm DB-HT Sim Dis column (Agilent) using a split mode (split ratio 2:1). The oven method employed was 30°C for 1 min, ramping at 7.5°C min⁻¹ to 100°C, followed by a ramp of 10°C min⁻¹ to 300°C and held at this temperature for 2 min. For peak identification, single hydrocarbons and two alkane standard solutions the 1^{st} with C8 to C20 and the 2^{nd} with C21 to C40 (Sigma) were used. Hydrocarbon determination was confirmed using an Agilent 6890N GC fitted with a 5973 series inert mass spectrophotometer, a HP-5 column (12 m x 0.2 mm x 0.33 µm) (Hewlett-Packard) was used with an oven method of 60°C for 6 min, increasing by 10°C min⁻¹ to 300°C and holding for 10 min.

### Results

### Conversion of PE pyrolysis wax to PHA in shake flasks

All nine strains were capable of growing on PE pyrolysis wax and under the screening conditions (0.05 % PE pyrolysis wax and 0.25 g L⁻¹ ammonium chloride) two strains accumulated PHA (Table 1).

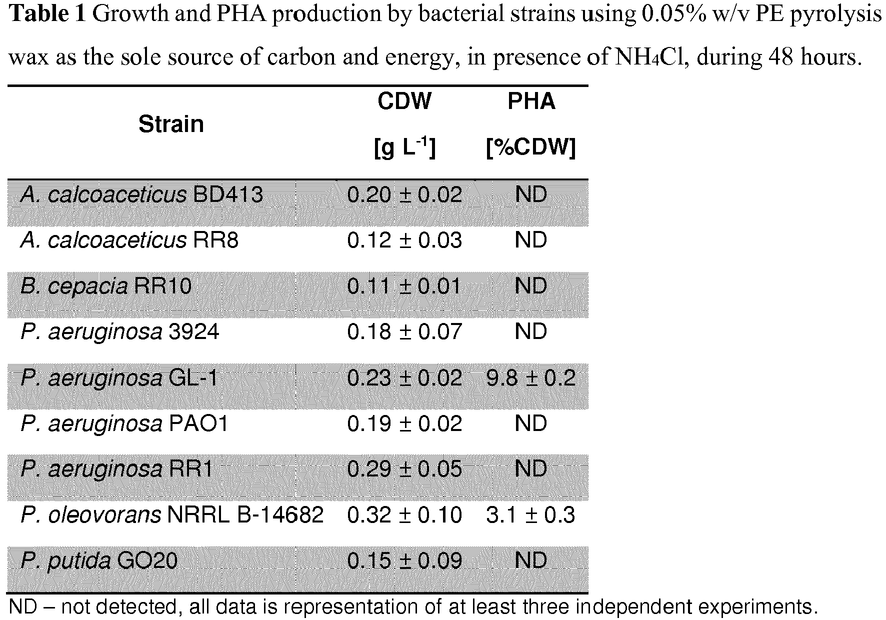

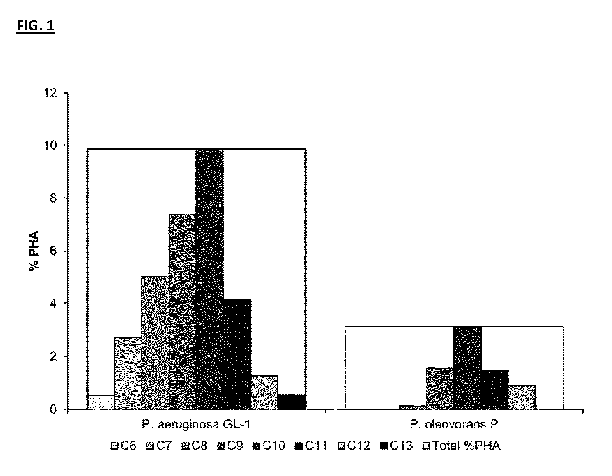

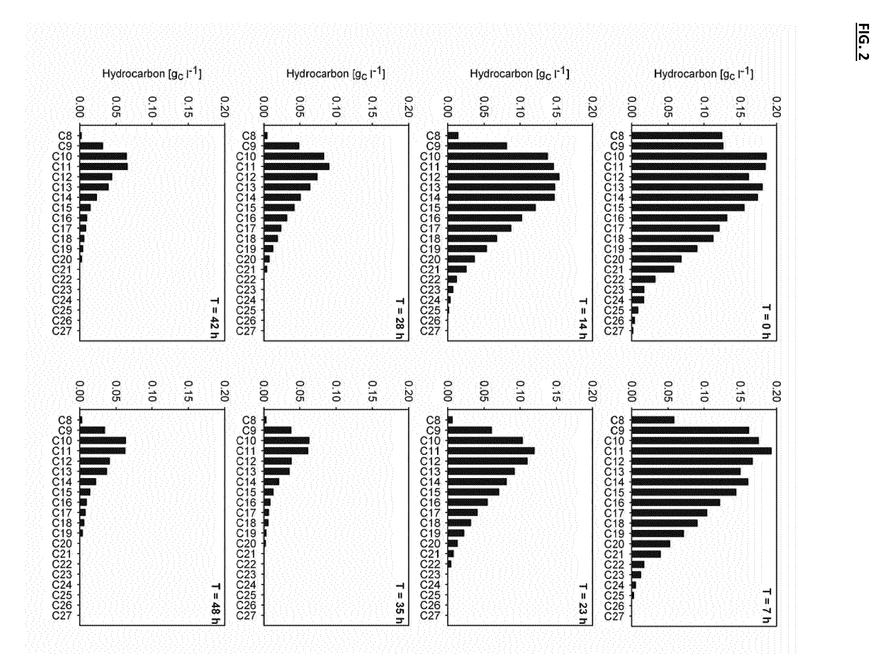

*P. aeruginosa* GL-1 accumulated 3 times more PHA than *P. aeruginosa PAO-1* (Table 1). Both strains accumulated mcl-PHA i.e. monomers with a carbon chain length ≥ 6 carbons. (*R*)-3-hydroxydecanoic acid was the predominant monomer accumulated by *P. aeruginosa* GL-1, with (*R*)-3-hydroxynonanoic acid as the second most prevalent monomer (Figure 1). PHA accumulated by strain PAO-1 contained monomers ranging from (*R*)-3-hydroxyheptanoic acid to (*R*)-3-hydroxydodecanoic acid, with (*R*)-3-hydroxydecanoic acid as the predominant monomer (Figure 2).
*P. aeruginosa* GL-1, which accumulated the most PHA from PE pyrolysis wax, was submitted for detailed PE pyrolysis wax hydrocarbon utilisation studies in shake flasks. Growth on PE pyrolysis wax was characterised by a lag period of 21 hours. However 58.2% of the PE pyrolysis wax substrate was removed from the growth media by that time. During a 48 h experiment this strain utilised 84.4% of the hydrocarbons supplied (Figure 2), producing 0.23 g L⁻¹ of CDW of which 9.8% was mcl-PHA when grown in MSM medium with limited nitrogen to promote PHA accumulation. This corresponds to a yield of biomass to substrate (Yx/s) of 0.14 g gc⁻¹ and yield of PHA (Y_{PHA/S}) of 0.013 g gc⁻¹.

### Enhancing PE pyrolysis wax bioavailability

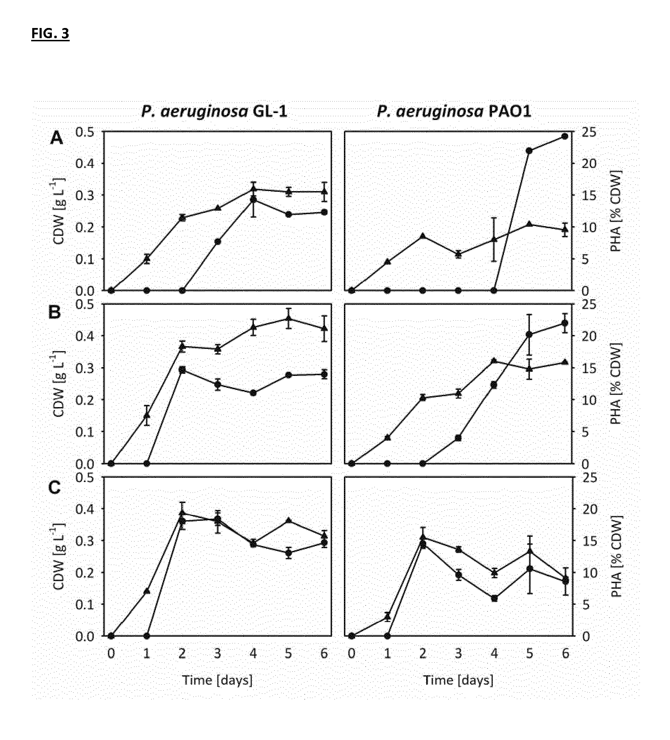

Increasing the PE pyrolysis wax concentration to 2% (w/v) did not result in better growth of *P. aeruginosa* GL-1 or PAO1 and no PHA accumulation was observed for either strain after 48 hours of growth (Figure 3A and 3B). *P. oleovorans* failed to grow with 2% (w/v) PE wax after 48 hours of incubation (data not shown). An extended incubation of strain GL-1 with 2% (w/v) PE wax resulted in similar growth but 1.6 fold more PHA accumulation (4 days) compared to growth with 0.05% wax (2 days) (Figure 3A). A dramatic improvement in PHA accumulation was observed for strain PAO1 with almost 25% of the cell dry weight as PHA (5 days) (Figure 3B). *P. oleovorans* failed to grow with 2% w/v PE pyrolysis wax even after 6 days of incubation (data not shown).
To further improve the PE wax to PHA process, the nitrogen source from ammonium chloride (NH₄Cl) to ammonium nitrite (NH₄NO₂) as the latter is known to increase surfactant (rhamnolipid) production in *P. aeruginosa* GL-1, which could aid growth on long chain hydrocarbons (Arino, *et al.,* 1998). For both strains, this inorganic nitrogen source enhanced the growth and triggered PHA accumulation one day earlier for strain GL-1 and 2 days earlier for PAO1 with 2% (w/v) PE wax. However the level of PHA accumulated (% CDW) did not increase compared to experiments with NH₄Cl. Finally, exogenous rhamnolipids were added to the liquid media in order to further enhance growth and PHA accumulation. The addition of 0.05% rhamnolipids to the culture media resulted in maximum biomass and PHA accumulation in strain PAO1 after 2 days of incubation (Figure 3C) which was an improvement, compared to cells grown in the absence of rhamnolipid where no PHA accumulation occurred after 2 days of growth (Figure 3B and 3C). Monomer composition of PHA was not affected by the change of nitrogen source or the presence of rhamnolipids (data not shown).
All microorganisms which were able to grow on PE pyrolysis wax (0.05% w/v, NH₄Cl), but failed to produce PHA (Table 2), were retested at PE pyrolysis wax concentration of 2% w/v, ammonium nitrate as the inorganic nitrogen source, and in the presence of 0.05% rhamnolipids Four strains showed not only improvement in biomass but were able to accumulate PHA (Table 3). The two *A*. *calcoaceticus* strains, namely BD413 and RR8, improved biomass levels 1.2 and 2.7 fold respectively and both accumulated low amounts of PHA (from 2.2 to 4.1% CDW, respectively). *P. aeruginosa* RR1 improved CDW levels 1.5 fold and accumulated 5.8% of the cell dry weight as PHA. *B. cepacia* RR10 acheived 3.1 fold higher biomass under the new growth conditions and accumulated 6.7% (CDW) PHA. None of the strains grew in control flasks where rhamnolipid, but no PE pyrolysis wax, was supplied alone to the growth medium. The composition of PHA was very similar to that isolated from strains GL-1 and PAO-1 with (R)-3-hydroxydecanoic acid as the predominant monomer but both even and uneven carbon chain monomers appearing in the PHA (data not shown).

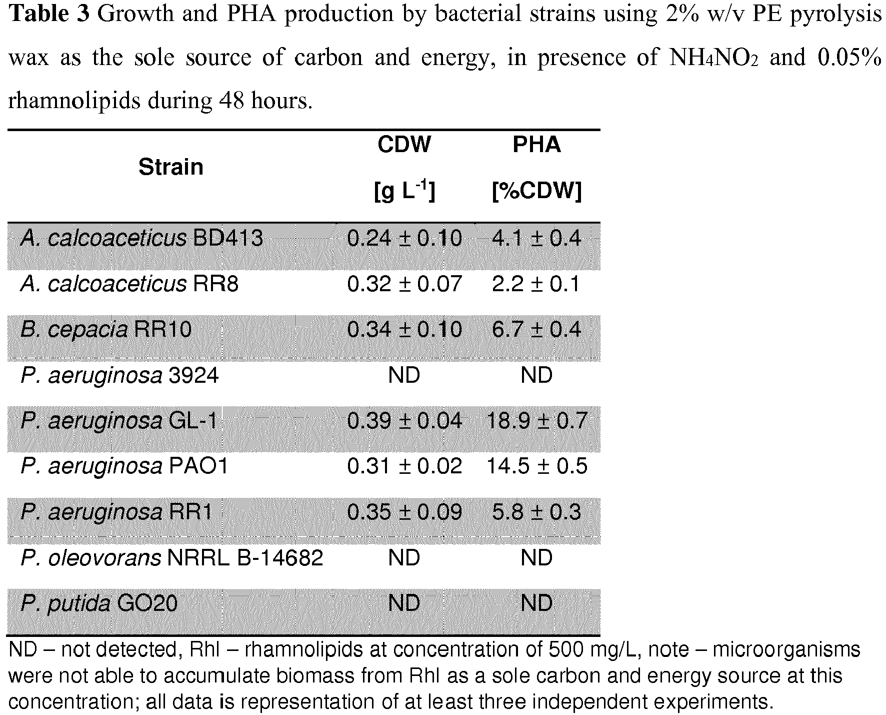

The invention is not limited to the embodiments hereinbefore described which may be varied in construction and detail without departing from the spirit of the invention.

### References

Aguado J, Serrano DP, Escola JM & Peral A (2009) Catalytic cracking of polyethylene over zeolite mordenite with enhanced textural properties. Journal of Analytical and Applied Pyrolysis 85: 352-358.
Al-Salem SM, Lettieri P & Baeyens J (2009) Recycling and recovery routes of plastic solid waste (PSW): a review. Waste Management 29: 2625-2643.
Arino S, Marchal R & Vandecasteele JP (1998) Involvement of a rhamnolipid-producing strain of Pseudomonas aeruginosa in the degradation of polycyclic aromatic hydrocarbons by a bacterial community. Journal of Applied Microbiology 84: 769-776.
Brandl H, Gross RA, Lenz RW & Fuller RC (1988) Pseudomonas oleovorans as a source of poly(β-hydroxyalkanoates) for potential applications as biodegradable polyesters. Applied and Environmental Microbiology 54: 1977-1982.
Butler E, Devlin G & McDonnell K (2011) Waste Polyolefins to Liquid Fuels via Pyrolysis: Review of Commercial State-of-the-Art and Recent Laboratory Research. Waste and Biomass Valorization 2: 227-255.
Conesa JA, Font R, Marcilla A & Garcia AN (1994) Pyrolysis of Polyethylene in a Fluidized Bed Reactor. Energy & Fuels 8: 1238-1246.
Hori K, Marsudi S & Unno H (2002) Simultaneous production of polyhydroxyalkanoates and rhamnolipids by Pseudomonas aeruginosa. Biotechnol Bioeng 78: 699-707.
Juni E & Janik A (1969) Transformation of Acinetobacter calcoaceticus (Bacterium anitratum). J. Bacteriol. 98: 281-288.
Kenny ST, Runic JN, Kaminsky W, et al. (2008) Up-Cycling of PET (Polyethylene Terephthalate) to the Biodegradable Plastic PHA (Polyhydroxyalkanoate). Environmental Science & Technology 42: 7696-7701.
Kumar S, Panda AK & Singh RK (2011) A review on tertiary recycling of high-density polyethylene to fuel. Resources, Conservation and Recycling 55: 893-910.
Lageveen RG, Huisman GW, Preusting H, Ketelaar P, Eggink G & Witholt B (1988) Formation of Polyesters by Pseudomonas oleovorans: Effect of Substrates on Formation and Composition of Poly-(R)-3-Hydroxyalkanoates and Poly-(R)-3-Hydroxyalkenoates. Appl Environ Microbiol 54: 2924-2932.
Rasul Jan M, Shah J & Gulab H (2010) Degradation of waste High-density polyethylene into fuel oil using basic catalyst. Fuel 89: 474-480..
Sabirova JS, Ferrer M, Lünsdorf H, et al. (2006) Mutation in a "tesB-Like" hydroxyacyl-coenzyme A-specific thioesterase gene causes hyperproduction of extracellular polyhydroxyalkanoates by Alcanivorax borkumensis SK2. Journal of Bacteriology 189: 289-290.
Scheiner D (1976) Determination of ammonia and Kjeldahl nitrogen by indophenol method. Water Research 10: 31-36.
Schlegel HG, Kaltwasser H & Gottschalk G (1961) A submersion method for culture of hydrogen-oxidizing bacteria: growth physiological studies. Archives of Microbiology 308: 209-222.
Stover CK, Pham XQ, Erwin AL, et al. (2000) Complete genome sequence of Pseudomonas aeruginosa PAO1, an opportunistic pathogen. Nature 406: 959-964.
Wallis MD & Bhatia SK (2007) Thermal degradation of high density polyethylene in a reactive extruder. Polymer Degradation and Stability 92: 1721-1729.
Ward PG, Goff M, Donner M, Kaminsky W & O'Connor KE (2006) A two step chemo-biotechnological conversion of polystyrene to a biodegradable thermoplastic. Environmental Science and Technology 40: 2433-2437.
Yakimov MM, Golyshin PN, Lang S, Moore ERB, Abraham W-R, Lünsdorf H & Timmis KN (1998) Alcanivorax borkumensis gen. nov., sp. nov., a new, hydrocarbon-degrading and surfactant-producing marine bacterium. International Journal of Systematic and Evolutionary Microbiology 48: 339-348
Yuste L, Corbella MaE, Turiégano MaJ, Karlson U, Puyet A & Rojo F (2000) Characterization of bacterial strains able to grow on high molecular mass residues from crude oil processing. FEMS Microbiology Ecology 32: 69-75.

## Claims

1. A method for producing polyhydroxyalkanoate (PHA), comprising the step of:
- culturing in a culture medium comprising pyrolysis wax obtained from the pyrolysis of polyethylene and optionally a rhamnolipid one or more bacterial strains which are capable of accumulating PHA from pyrolysis wax; and
- recovering the PHA from the culture medium.

2. A method as claimed in Claim 1 in which the bacterial strains are selected from the group consisting of: *A*. *calcoaceticus* BD413; A. *calcoaceticus* RR8; *B. sepacia* RR10; *P. aeruginosa* 3924; *P. aeruginosa* GL-1; *P. aeruginosa* PAO1; *P. aeruginosa* RR1; and *P. olevorans.*

3. A method as claimed in Claim 1 in which the bacterial strains are selected from *P*. *aeruginosa* 3924 and *P. olevorans.*

4. A method as claimed in Claim 1 in which the culture medium comprises a rhamnolipid in addition to the pyrolysis wax, and wherein the bacterial strains are selected from the group consisting of: A. *calcoaceticus* BD413; *A*. *calcoaceticus* RR8; *B. sepacia* RR10; *P. aeruginosa* GL-1; *P. aeruginosa* PAO1; and *P*. *aeruginosa* RR1.

5. A method as claimed in Claim 4 in which the culture medium comprises 0.01% to 0.1% rhamnolipid (w/v).

6. A method as claimed in any preceding Claim, in which the culture medium comprises an inorganic nitrogen source.

7. A method as claimed in any preceding Claim in which the culture medium comprises 0.01% to 5% pyrolysis wax (w/v).

8. A method as claimed in Claim 7 in which the culture medium comprises 1% to 3% pyrolysis wax (w/v).

9. A method as claimed in Claim 1 in which the culture medium comprises 0.01% to 5.0% pyrolysis wax (w/v), an ammonium salt in an amount of 0.1% to 1.0%, and optionally a rhamnolipid in an amount of 0.01% to 0.1% (w/v).

10. A method as claimed in Claim 9 in which the culture medium comprises 0.01% to 5.0% pyrolysis wax (w/v), and an ammonium salt in an amount of 0.1% to 1.0%, in which the bacterial strains are selected from *P. aeruginosa* 3924 and *P. olevorans.*

11. A method as claimed in Claim 1 in which the culture medium comprises 0.01 % to 5.0% pyrolysis wax (w/v), an ammonium salt in an amount of 0.1% to 1.0%, and a rhamnolipid in an amount of 0.01% to 0.1% (w/v), and wherein the bacterial strains are selected from the group consisting of: A. *calcoaceticus* BD413; A. *calcoaceticus* RR8; *B. sepacia* RR10; *P. aeruginosa* GL-1; *P. aeruginosa* PAO1; and *P. aeruginosa* RR1.

12. A method for producing polyhydroxyalkanoate (PHA) from polyethylene (PE), comprising the steps of:
- subjecting the polyethylene to pyrolysis under suitable conditions for producing a liquid diesel fraction and a pyrolysis was fraction, and
- producing PHA from the pyrolysis according to a method of any of Claims 1 to 11.

13. PHA obtained according to a method of any of Claims 1 to 12.
